(19) Europäisches Patentamt
European Patent Office
Office européen des brevets

(11) Veröffentlichungsnummer: **0 005 465**
**B1**

# (12) EUROPÄISCHE PATENTSCHRIFT

(45) Veröffentlichungstag der Patentschrift: **25.11.81**

(21) Anmeldenummer: **79101256.0**

(22) Anmeldetag: **26.04.79**

(51) Int. Cl.³: **C 07 D 405/14**, D 06 L 3/12, C 08 K 5/34, D 21 H 3/80 // (C07D405/14, 249/04, 307/78, 235/04)

## (54) Benzofuranyl-benzimidazole, Verfahren zu ihrer Herstellung sowie ihre Verwendung zum optischen Aufhellen von organischen Materialien.

(30) Priorität: **13.05.78 DE 2821116**

(43) Veröffentlichungstag der Anmeldung: **28.11.79 Patentblatt 79/24**

(45) Bekanntmachung des Hinweises auf die Patenterteilung: **25.11.81 Patentblatt 81/47**

(84) Benannte Vertragsstaaten: **BE CH DE FR GB IT**

(56) Entgegenhaltungen:
**DE - A 2 159 469**
**DE - A 2 166 632**
**DE - A 2 522 139**

(73) Patentinhaber: **BAYER AG, Zentralbereich Patente, Marken und Lizenzen, D-5090 Leverkusen 1, Bayerwerk (DE)**

(72) Erfinder: **Bremen, Josef, Dr., Am Kettnersbusch 1e,, D-5090 Leverkusen 3 (DE)**
Erfinder: **Wehling, Bernhard, Dr., Andreas-Gryphius-Strasse 26, D-5000 Köln 80 (DE)**
Erfinder: **Schellhammer, Carl-Wolfgang, Dr., Katharinenthal 26, D-5060 Bergisch-Gladbach (DE)**

EP 0 005 465 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Benzofuranyl-benzimidazole, Verfahren zu ihrer Herstellung sowie ihre Verwendung zum optischen Aufhellen von organischen Materialien

Gegenstand der Erfindung sind Benzofuranyl-benzimidazole der allgemeinen Formel

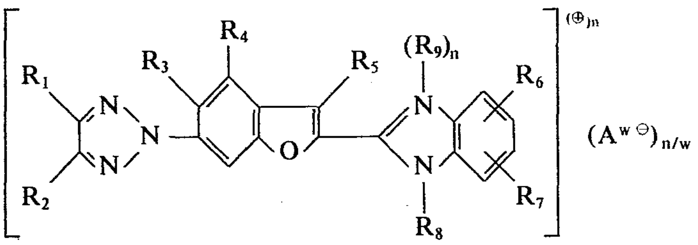

(I)

in der

$R_1$ eine Alkylgruppe mit 1 bis 12 Kohlenstoffatomen, eine Alkoxygruppe mit 1–4 Kohlenstoffatomen, einen Phenylrest, der gegebenenfalls durch Alkyl mit 1–4 C-Atomen, Phenyl, Alkoxy mit 1–4 C-Atomen oder Chlor substituiert sein kann, einen Benzylrest oder einen Styrylrest,

$R_2$ Wasserstoff, Cyan, Carboxy, $C_1$–$C_4$-Alkylcarbonylamino, Benzoylamino oder den Rest R oder

$R_1$ und $R_2$ zusammen einen ankondensierten Naphthalinring oder Benzolring, der gegebenenfalls durch Alkyl mit 1 bis 4 Kohlenstoffatomen und/oder Alkoxy mit 1 bis 4 Kohlenstoffatomen substituiert sein kann,

$R_3$ und $R_4$ unabhängig voneinander Wasserstoff, Methyl, Äthyl, Methoxy, Chlor,

$R_5$ Wasserstoff, Alkyl mit 1 bis 4 Kohlenstoffatomen, vorzugsweise Methyl, oder gegebenenfalls mit Methyl und/oder Methoxy substituiertes Phenyl,

$R_6$ Wasserstoff, Alkyl mit 1 bis 4 Kohlenstoffatomen, vorzugsweise Methyl, Methoxy, Chlor, Alkylsulfonyl mit 1 bis 4 Kohlenstoffatomen, Cyan, eine Sulfo- oder Carboxygruppe oder deren funktionelle Derivate,

$R_7$ Wasserstoff, Methyl, Methoxy oder Cl,

$R_8$ Alkyl mit 1 bis 4 Kohlenstoffatomen, vorzugsweise Methyl, Hydroxyalkyl mit 2 bis 4 Kohlenstoffatomen, Cyanethyl, Cyclohexyl, Benzyl oder gegebenenfalls durch Chlor, Methyl oder Methoxy substituiertes Phenyl,

$R_9$ gegebenenfalls mit Hydroxy oder Alkoxy mit 1 bis 4 Kohlenstoffatomen substituiertes Alkyl mit 1 bis 4 Kohlenstoffatomen, gegebenenfalls mit Chlor oder Methoxy substituiertes Benzyl oder einen Rest $-CH_2CN$, $-CH_2CONH_2$ oder $-CH_2COOR$, worin R eine Alkylgruppe mit 1 bis 4 Kohlenstoffatomen, vorzugsweise Methyl, ist,

n die Zahl 0 oder 1,

$A^{\ominus}$ ein farbloses Anion, das auf die Aufhellereigenschaften der Verbindungen (I) keinen wesentlichen Einfluß hat, wie z. B. das Halogenid-, Formiat-, Acetat-, Lactat-, $CH_3SO_4^{\ominus}$-, $C_2H_5SO_4^{\ominus}$-, $C_6H_5SO_3^{\ominus}$-, p-$CH_3C_6H_4SO_3^{\ominus}$-, Carbonat- oder Bicarbonation und

w die Wertigkeit des Anions A

bedeuten.

Im Rahmen der Verbindungen der Formel (I) sind solche Verbindungen bevorzugt, die der Formel

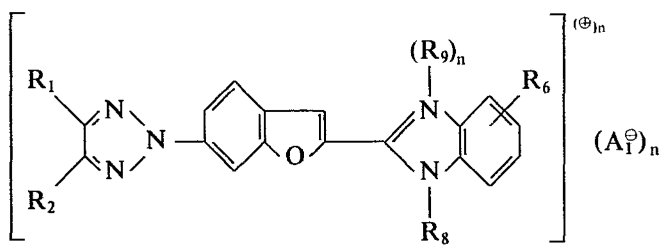

(II)

entsprechen, worin

$R_1$, $R_2$, $R_6$, $R_8$ und $R_9$ die oben angegebene Bedeutung haben,

n die Zahl 0 oder 1 und

$A_1^{\ominus}$ ein Halogen-, Methylsulfat-, Ethylsulfat- oder p-Toluylsulfation bedeuten.

Die neuen Verbindungen der Formel (I) erhält man, indem man eine Verbindung der Formel

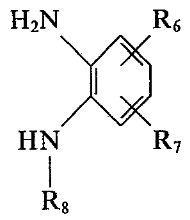

(III)

in der

$R_6$, $R_7$, $R_8$ die oben angegebene Bedeutung haben,

mit einer Verbindung der Formel

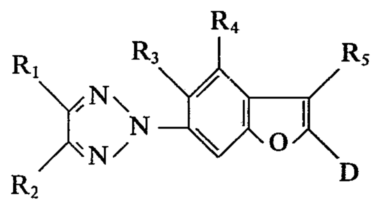

(IV)

in der

$R_1$—$R_5$ die oben angegebene Bedeutung haben, und
D COOH oder eine funktionelle abgewandelte Carboxylgruppe bedeutet,

acyliert und in dem Acylierungsprodukt in an sich bekannter Weise den Imidazolringschluß herbeiführt.

Dieser Ringschluß wird bevorzugt in Gegenwart saurer Kondensationsmittel, wie z. B. Essigsäure, Chlorwasserstoff, Borsäure, Zinkchlorid, Polyphosphorsäure, Phosphorsäure oder p-Toluolsulfonsäuren, durchgeführt. Die erhaltenen Verbindungen werden gegebenenfalls in bekannter Weise quaterniert oder mit einer Säure protoniert.

Bei Verbindungen der Formel (I), die einem $SO_3H$-Rest enthalten, kann die stark saure Sulfogruppe mit dem basischen Imidazolring ein inneres Salz bilden. Die Gruppe $SO_3^{\ominus}$ als einer der Substituenten des Benzimidazolringes kann in quaternierten oder protonierten Verbindungen der Formel (I), also auch als Anion $A^{\ominus}$ fungieren.

Zu Verbindungen der Formel (I), worin n für null steht (nicht quaternierte Verbindungen), gelangt man auch, indem man am Imidazolring unsubstituierte Verbindungen der Formel (I), worin $R_8$ Wasserstoff und n null bedeutet, mit Alkylierungsmitteln in Gegenwart von basisch wirkenden Verbindungen nach bekannten Verfahren umsetzt.

Geeignete Derivate der Carbonsäuren der Formel (IV) sind deren Salze, Halogenide, Ester, Amide, Iminoether sowie Nitrile.

Geeignete Alkylierungsmittel sind Dialkylsulfat, Alkylhalogenide, Alkylbenzolsulfonate, Benzylhalogenide sowie $BrCH_2CH_2OH$, $BrCH_2CHOHCH_3$, $ClCH_2CO_2CH_2CH_3$, $BrCH_2COOH$, $BrCH_2COOCH_3$, $ClCH_2CN$, $ClCH_2CONH_2$, $ClCH_2CONHCH_3$ und $ClCH_2CON(CH_3)_2$ sowie Ethylenoxid oder Propylenoxid in Gegenwart geeigneter Anionen, wie z. B. der Ameisen-, Essig- oder Milchsäure.

Geeignete Protonierungsmittel sind starke bis mittelstarke organische Säuren oder Mineralsäuren.

Alternativ können die Verbindungen der Formel (I) auch hergestellt werden, indem man ein o-Nitroanilin der Formel

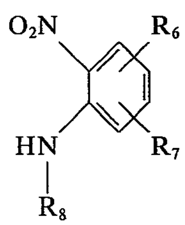

(VI)

mit einer Verbindung der Formel (IV) acyliert, die Nitrogruppe in saurem Medium reduziert und gleichzeitig den Ringschluß zum Imidazolring herbeiführt.

Die Ausgangsprodukte der Formel (V) werden in an sich bekannter Weise durch Umsetzung von o-Chlor-nitrobenzol-derivaten mit primären Aminen oder Ammoniak zu den entsprechend substituierten o-Nitroanilinen und Reduktionen der letzteren hergestellt (vgl. BE-PS 595 327, DE-OS 2 239 614 und DE-OS 1 522 412).

Die Ausgangsverbindungen der Formel (IV) können hergestellt werden:

1) durch Cumarilsäureumlagerung (vgl. Krauch-Kunz 3. Aufl. Reaktionen der organischen Chemie, S. 449) aus den Cumarinderivaten der Formel

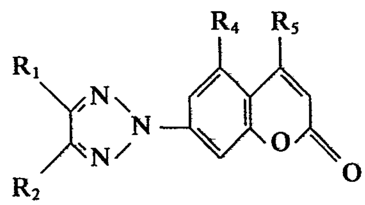

(VI)

oder

2) durch Umsetzung von Verbindungen der Formel

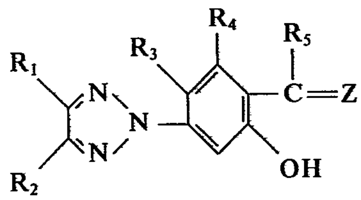

(VII)

worin

Z o oder N—R bedeutet, wobei

R einen gegebenenfalls substituierten Phenylrest bedeutet, mit Halogenmalonestern oder Formel

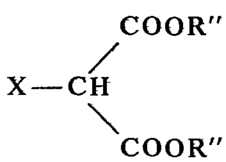

(VIII)

worin

X Chlor, Brom oder Jod und

R″ eine Alkylgruppe bedeuten,

in an sich bekannter Weise [vgl. S. Tanaka, J. Am. Chem. Soc. 73, S. 872 (1951)], wobei zunächst die Verbindung der Formel

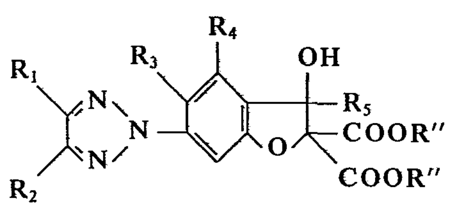

(IX)

entsteht, die dann in an sich bekannter Weise durch Verseifung, Dehydratisierung und Decarboxylierung in die Verbindung der Formel (IV) übergeführt wird,

3) durch Umsetzung der Verbindungen der Formel (VII) mit Halogenessigestern der Formel

$$Z—CH_2—COOR^x \qquad (X)$$

worin

Z Chlor, Brom oder Jod, insbesondere Chlor oder Brom und

$R^x$ eine Alkylgruppe bedeuten,

in an sich bekannter Weise (vgl. Bull. Soc. Chim. France 1971, S. 4329 bis 4331), wobei die Umsetzung zweckmäßigerweise in Lösung der Gegenwart säurebindender Substanzen durchgeführt wird,

4) durch Cyclisierung von Oximinohydrazonen der Formel

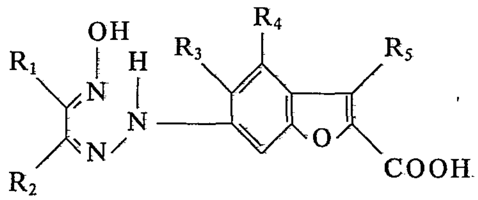

(XI)

nach bekannten Verfahren (vgl. Deutsche Offenlegungsschrift 1 670 914, Britische Patentschrift 1 154 995).

(Die Oximinohydrazone der Formel (XI) werden beispielsweise aus 6-Nitrobenzofurancarbonsäurederivaten der Formel

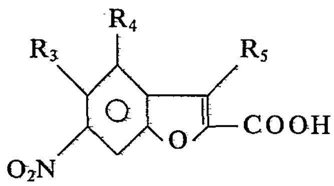

(XII)

durch Reduktion der Nitrogruppe, Diazotieren der erhaltenen Aminogruppe, Reduktion der Diazoniumgruppe zur Hydrazinogruppe und Umsetzung mit -Oximinoketonen der Formel

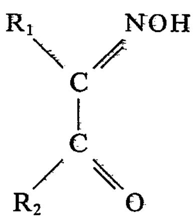

(XIII)

hergestellt.)

5) durch thermische Decarboxylierung und Umlagerung der Azofarbstoffe der Formel

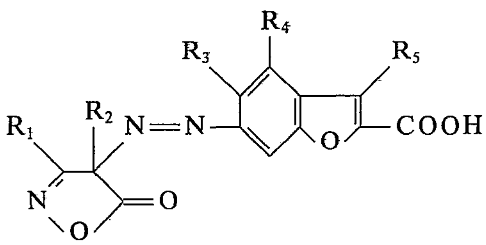

(XIV)

die bei der Kupplung der Diazoniumsalze der Formel

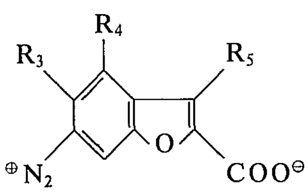

(XV)

auf in 3- und 4-Stellung substituierte Isoxazolin-5-one der Formel

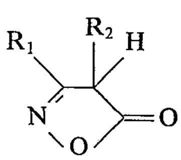

(XVI)

entstehen.

6) Schließlich können die Verbindungen der Formel (IV), in der $R_1$ und $R_2$ zusammen einen anellierten aromatischen Ring der obengenannten Weise bedeuten, durch Kuppeln des Diazoniumsalzes der Formel (XV) auf ein primäres aromatisches Amin aus der Benzol- oder Naphthalinreihe zu einer o-Aminoazoverbindung der Formel

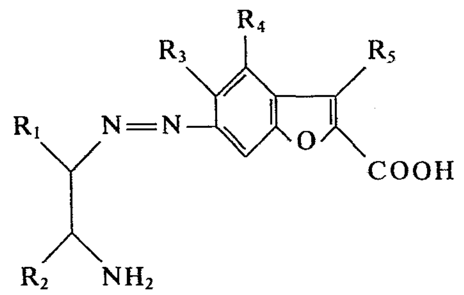

(XVII)

worin
$R_1$ und $R_2$ ein anelliertes aromatisches Ringsystem bedeuten,
und anschließender Oxidation zum Triazolringsystem nach bekannten Methoden (vgl. Deutsche Offenlegungsschrift 1 519 471) hergestellt werden.

Die erfindungsgemäßen Verbindungen der Formel (I) zeigen in gelöstem oder feinverteiltem Zustand eine ausgeprägte Fluoreszenz. Sie eignen sich zum optischen Aufhellen der verschiedensten organischen Materialien, vor allem zum optischen Aufhellen von Fasern, Fäden, Geweben, Gewirken oder Folien aus z. B. Polyestern, Polyamiden, Celluloseestern und insbesondere aus Polyacrylnitril.

Vorzugsweise wird farbloses, hochmolekulares organisches Material in Form von Fasern nach an sich bekannten Verfahren aufgehellt.

Erfindungsgemäß optisch aufgehelltes Material weist ein gefälliges, rein weißes, blauviolett bis blaustichig fluoreszierendes Aussehen auf.

Waschflotten, die Benzofurane der Formel (I) enthalten, verleihen beim Waschen den damit behandelten Textilfasern, insbesondere Polyacrylnitrilfasern, einen brillanten Aspekt im Tageslicht. Die erzielten Aufhellungen sind lichtecht und waschbeständig.

Diese Effekte müssen als überraschend bezeichnet werden, da aus der DE-A 2 159 469 nur solche Benzofuranylbenzimidazole bekannt waren, die in 6-Stellung des Benzofuranrestes sehr »kleine« Substituenten, wie Wasserstoff, Chlor und niedriges Alkoxy, tragen, so daß erwartet werden konnte, daß durch Einführung des größeren und häufig fluoreszenzverstärkenden 1,2,3-Triazolyl-2-Restes in jene 6-Stellung deutlich schwerer lösliche und damit weniger ergiebige Produkte entstehen würden, die bereits im sichtbaren Licht absorbieren.

## Beispiel 1

In eine Lösung von 2,7 g N-Methyl-o-phenylendiamin in 60 ml Pyridin werden bei Raumtemperatur innerhalb von 5 Minuten 7,0 g 6-(Naphth—[1,2-d]1,2,3-triazol-2-yl)-benzofuran-2-carbonsäurechlorid eingetragen. Es wird 15 Minuten nachgerührt und dann 6 Stunden bei 80—85°C gerührt. Hierbei fällt das 6-(Naphth—[1,2-d]1,2,3-triazol-2-yl)-benzofuran-2-carbonsäure-N-methyl-o-aminoanilid in gelblichen weißen Kristallen aus. Nach dem Abkühlen auf 5°C wird abgesaugt und mit wenig Pyridin nachgewaschen. Der Filterrückstand wird in 200 ml Wasser suspendiert und die Suspension mit 10%iger Salzsäure auf pH 2—3 gestellt. Hierauf wird abgesaugt und mehrmals mit Wasser gewaschen.

Das vorstehend erhaltene Acylierungsprodukt wird noch feucht in 150 ml Glykolmonomethyläther bei 80°C suspendiert. Man versetzt mit 10 ml konzentrierter Salzsäure, wobei sich das Produkt augenblicklich auflöst. Es wird 1 ½ Stunden unter Rückfluß gerührt. Hierbei kristallisiert allmählich das Benzimidazol der Formel

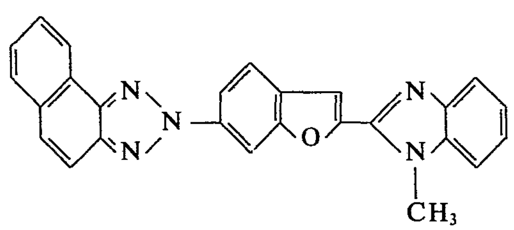

(101)

als Hydrochlorid aus. Man kühlt auf 5°C ab. Das erhaltene Produkt wird zur Überführung in die freie Base in 150 ml Methanol suspendiert und bei 50°C mit 5 ml Triäthylamin versetzt. Es wird eine Stunde bei dieser Temperatur gerührt, abgesaugt, mit Methanol gewaschen und getrocknet. Durch Umkristallisation aus Dimethylformamid erhält man 6,1 g (73% der Theorie) der Verbindung der Formel (101) in gelblichen Kristallen, welche in Lösung blau-violett fluoreszieren.

Das als Ausgangsprodukt verwendete 6-(Naphth[1,2-d]1,2,3-triazol-2-yl)-benzofuran-2-carbonsäurechlorid wird wie folgt hergestellt:

Eine Suspension aus
28,9 g 4-(Naphth–[1,2-d]1,2,3-triazol-2-yl)-salicylaldehyd,
31,3 g Brommalonsäurediäthylester und
20,7 g Kaliumcarbonat in 400 ml Dimethylsulfoxid wird bei 80°C 2 Stunden gerührt. Hierauf wird das Lösungsmittel im Vakuum abdestilliert, der Rückstand mit 400 ml Äthanol verrührt, mit 20%iger Schwefelsäure angesäuert, abgesaugt und mit Wasser gewaschen. Der Filterkuchen wird in 500 ml Äthanol, die 22,4 g 50%ige Kaliumlauge enthalten, 6 Stunden gekocht. Hierauf wird das Lösungsmittel im Vakuum abgedampft und der Rückstand mit 500 ml Wasser verrührt. Man stellt mit konzentrierter Salzsäure kongosauer und saugt ab. Nach Umkristallisation aus Äthylenglykolmonomethyläther unter Zuhilfenahme von Tonsil erhält man 21,1 g (64% der Theorie) 6-(Naphth[1,2-d]1,2,3-triazol-2-yl)-benzofuran-2-carbonsäure mit einem Schmelzpunkt von >300°C.

8,0 g der so hergestellten Benzofuran-2-carbonsäure werden in 10 g Thionylchlorid, 250 ml Toluol und 0,5 ml Dimethylformamid 2 Stunden unter Rückfluß erhitzt. Hierauf wird auf 0°C abgekühlt, abgesaugt, aus Toluol umkristallisiert und getrocknet. Man erhält 7,7 g (91% der Theorie) 6-(Naphth-[1,2-d]-1,2,3-triazol-2-yl)-benzofuran-2-carbonsäurechlorid in gelblichen Kristallen, welche bei 230 bis 231°C schmelzen.

## Beispiel 2

3,0 g der Verbindung der Formel (101) werden in 150 ml 1,2-Dichlorbenzol durch Erwärmen auf 140°C gelöst. In die erhaltene Lösung werden bei 120°C 2,0 ml Dimethylsulfat getropft. Nach beendigtem Zutropfen wird 3 Stunden bei 130–140°C gerüht. Hierbei fällt allmählich das quaternäre Benzimidazol aus. Man läßt auf 90–100°C abkühlen, saugt ab, wäscht mehrmals mit heißem 1,2-Dichlorbenzol nach und trocknet bei 100°C im Ölpumpenvakuum. Man erhält 3,8 g (97% der Theorie) des quaternären Benzofuranyl-benzimidazols der Formel

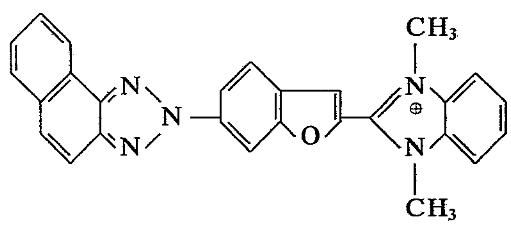

$CH_3OSO_3^{\ominus}$ (201)

in Form gelblicher Kristalle.

Die Verbindung löst sich in Wasser mit blauer Fluoreszenz am Tageslicht und ist vorzüglich geeignet zum Aufhellen von Polyacrylnitrilfasern.

## Beispiel 3

In eine Lösung von 2,7 g N-Methyl-o-phenylendiamin in 60 ml Pyridin werden bei Raumtemperatur innerhalb von 5 Minuten 6,8 g 6-(4-Methyl-5-phenyl-1,2,3-triazol-2-yl)-benzofuran-2-carbonsäurechlorid eingetragen. Hierbei steigt die Temperatur auf 35–40°C an. Es wird 15 Minuten nachgerührt und dann 6 Stunden bei 80–85°C gerührt. Es wird auf Raumtemperatur abgekühlt und in 600 ml Wasser gegossen. Die Mischung wird mit 30 ml konzentrierter Salzsäure versetzt und 30 Minuten bei Raumtemperatur gerührt. Es wird ein kristalliner Niederschlag erhalten. Man saugt ab, wäscht mehrmals mit 5%iger wäßriger Salzsäure nach und cyclisiert das noch feuchte Zwischenprodukt in Äthylenglykolmonomethyläther, wie in Beispiel 1 zur Herstellung des Benzimidazols der Formel (101) beschrieben. Es werden 5,2 g (64% der Theorie) des Benzimidazols der Formel

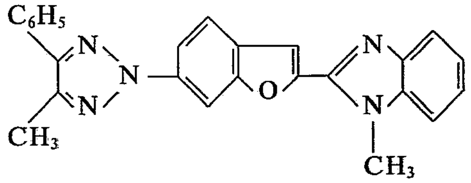

(301)

in gelblichen Kristallen erhalten, welche in Lösung blau-violett fluoreszieren.

Verwendet man anstelle von N-Methyl-o-phenylendiamin die äquivalenten Mengen 2-Methylamino-5-methylsulfonyl-anilin bzw. 2-Methylamino-5-methyl-anilin bzw. 2-Methylamino-5-chloranilin und verfährt im übrigen wie vorstehend beschrieben, so erhält man die Benzimidazole der Formeln

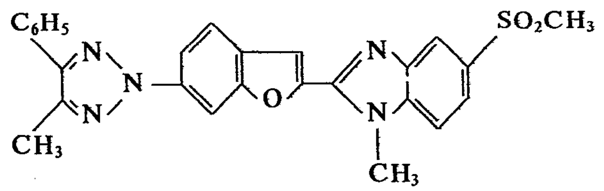

(302)

Fluoreszenzfarbe in Lösung: neutral blau
bzw.

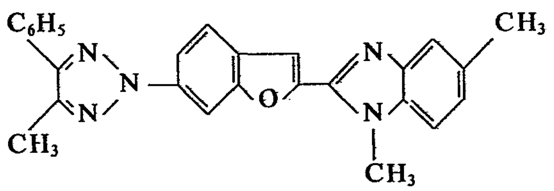

(303)

Fluoreszenzfarbe in Lösung: blau-violett
bzw.

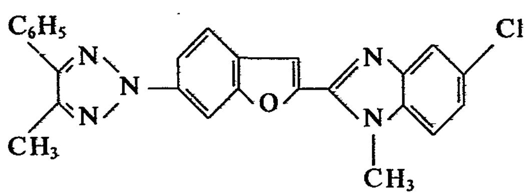

(304)

Fluoreszenzfarbe in Lösung: neutral blau.

Das als Ausgangsprodukt verwendete 6-(4-Methyl-5-phenyl-1,2,3-triazol-2-yl)-benzofuran-2-carbonsäurechlorid wird analog der in Beispiel 1 beschriebenen Herstellung von 6-(Naphth[1,2-d]-1,2,3-triazol-1-yl)-benzofuran-2-carbonsäurechlorid erhalten, wenn man den 4-(Naphth[1,2-d]-1,2,3-triazol-2-yl)-salicylaldehyd durch die äquivalente Menge 4-(4-Methyl-5-phenyl-1,2,3-triazol-2-yl)-salicylaldehyd ersetzt und im übrigen so verfährt, wie in Beispiel 1 beschrieben.

Die als Zwischenprodukt in 87% der Theorie erhaltene 6-(4-Methyl-5-phenyl-1,2,3-triazol-2-yl)-benzofuran-2-carbonsäure hat einen Schmelzpunkt von 245—248°C. Das hieraus in 90%iger Ausbeute hergestellte Säurechlorid schmilzt nach Umkristallisieren aus Cyclohexan bei 159—160°C.

## Beispiel 4

3,0 g der Verbindung der Formel (301) werden in 150 ml Chlorbenzol durch Erhitzen zum Rückfluß gelöst. In die Lösung werden bei 100°C 2,0 ml Dimethylsulfat getropft. Nach beendigtem Zutropfen wird 4 Stunden unter Rückfluß gerührt. Hierbei fällt das quaternäre Salz aus. Man läßt auf 90°C abkühlen, saugt ab, wäscht mehrmals mit heißem Chlorbenzol nach und trocknet bei 100°C im Ölpumpenvakuum. Man erhält 3,6 g (92% der Theorie) der Verbindung der Formel

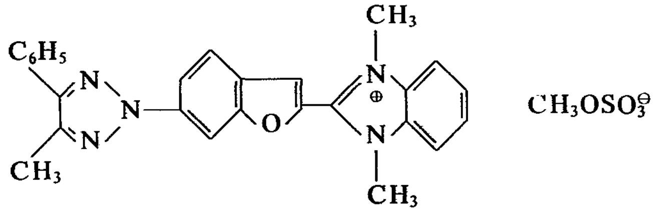

(401)

in Form gelber Kristalle. Die Verbindung löst sich in Wasser mit neutralblauer Fluoreszenz am Tageslicht.

Sie eignet sich vorzüglich zum Aufhellen von Polyacrylnitrilfasern.

Verwendet man anstelle der Verbindung der Formel (301) die gleichen Mengen der Verbindungen der Formeln (302) bzw. (303) bzw. (304) und verfährt im übrigen wie vorstehend beschrieben, so erhält man die quaternären Salze der Formeln

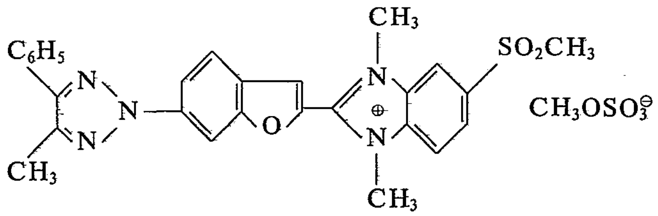

(402)

bzw.

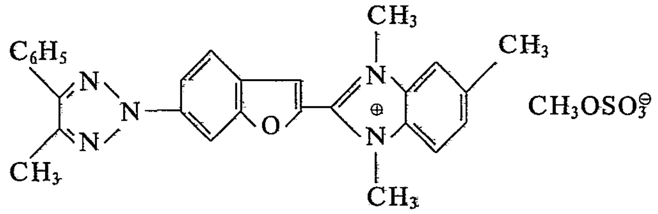

(403)

bzw.

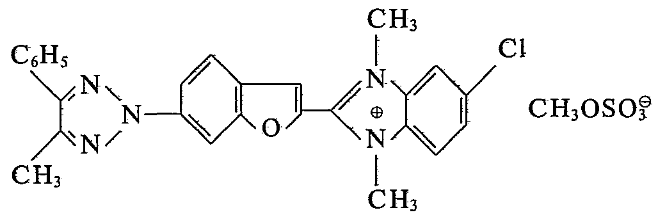

(404)

Diese Produkte besitzen ähnliche Eigenschaften wie die oben beschriebene Verbindung (401).

Beispiel 5

Verwendet man anstelle von 6,8 g 6-(4-Methyl-5-phenyl-1,2,3-triazol-2-yl)-benzofuran-2-carbonsäurechlorid die äquivalente Menge 6-(4-Phenyl-1,2,3-triazol-2-yl)-benzofuran-2-carbonsäurechlorid und verfährt im übrigen wie in Beispiel 3 beschrieben, so erhält man in 65% Ausbeute das Benzimidazol der Formel

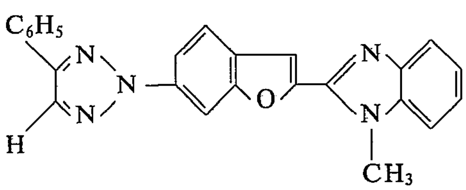

(501)

in gelblichen Kristallen, die in Lösung rot-violett fluoreszieren.

Auf analoge Weise gelangt man zu den in Tabelle 1 aufgeführten Verbindungen der Formel

Tabelle 1

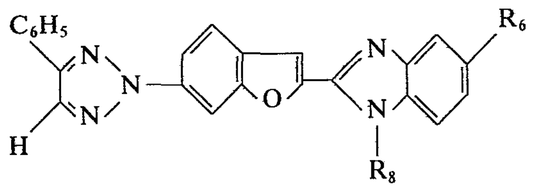

| Verbindung | $R_8$ | $R_6$ | Fluoreszenzfarbe in Lösung |
|---|---|---|---|
| 502 | $CH_3$ | $CH_3$ | rot-violett |
| 503 | $CH_3$ | Cl | blau-violett |
| 504 | $CH_3$ | $SO_2{-}CH_3$ | blau-violett |
| 505 | H | H | rot-violett |
| 506 | H | $CH_3$ | rot-violett |
| 507 | $CH_2{-}C_6H_5$ | H | rot-violett |
| 508 | $CH_2{-}C_6H_5$ | $CH_3$ | rot-violett |

Das als Ausgangsprodukt verwendete 6-(4-Phenyl-1,2,3-triazol-2-yl)-benzofuran-2-carbonsäurechlorid wird analog der in Beispiel 1 beschriebenen Herstellung von 6-(Naphth[1,2-d]-1,2,3-triazol-2-yl)-benzofuran-2-carbonsäurechlorid erhalten, wenn man den 4-(Naphth[1,2-d]-1,2,3-triazol-2-yl)-salicylaldehyd durch 4-(4-Phenyl-1,2,3-triazol-2-yl)-salicylaldehyd ersetzt und im übrigen so verfährt, wie dort beschrieben.

Die als Zwischenprodukt in 80% der Theorie erhaltene 6-(4-Phenyl-1,2,3-triazol-2-yl)-benzofuran-2-carbonsäure schmilzt bei 195—199°C. Das hieraus in 85%iger Ausbeute hergestellte Säurechlorid schmilzt nach Umkristallisieren aus Toluol bei 228—229°C.

Beispiel 6

Zur Herstellung der in Tabelle 2 aufgeführt quaternären Salze der Formel

Tabelle 2

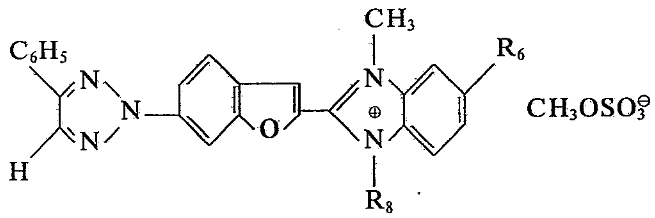

verfährt man analog, wie in Beispiel 4 beschrieben.

| Verbindung | $R_8$ | $R_6$ | Fluoreszenzfarbe in Lösung |
|---|---|---|---|
| 601 | $CH_3$ | H | blau-violett |
| 602 | $CH_3$ | $CH_3$ | blau-violett |
| 603 | $CH_3$ | Cl | blau |
| 604 | $CH_3$ | $SO_2-CH_3$ | neutral blau |
| 607 | $CH_2-C_6H_5$ | H | blau-violett |
| 608 | $CH_2-C_6H_5$ | $CH_3$ | blau-violett |

Beispiel 7

Auf analoge Weise, wie in den vorstehenden Beispielen beschrieben, gelangt man zu den in Tabelle 3 aufgeführten Verbindungen der Formel

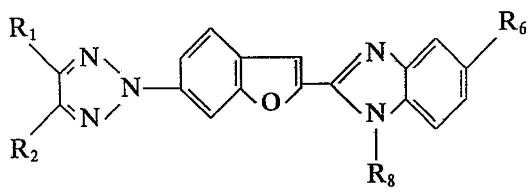

bzw. quaternären Verbindungen der Formel

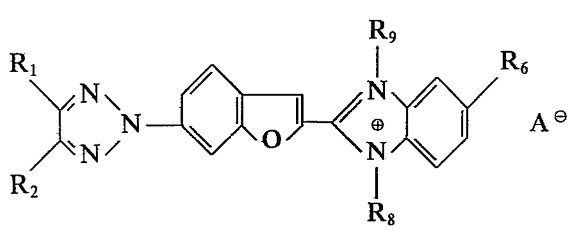

Tabelle 3

| Verbindung | $R_1$ | $R_2$ | $R_6$ | $R_8$ | $R_9$ | $A^{\ominus}$ | Fluoreszenzfarbe in Lösung |
|---|---|---|---|---|---|---|---|
| 701 | $C_2H_5$ | $CH_3$ | H | H | – | – | rotviolett |
| 702 | $C_2H_5$ | $CH_3$ | H | $CH_3$ | – | – | rotviolett |
| 703 | $C_2H_5$ | $CH_3$ | H | $CH_2-C_6H_5$ | – | – | rotviolett |
| 704 | $C_2H_5$ | $CH_3$ | $CH_3$ | H | – | – | rotviolett |
| 705 | $C_2H_5$ | $CH_3$ | $CH_3$ | $CH_3$ | – | – | rotviolett |
| 706 | $C_2H_5$ | $CH_3$ | Cl | H | – | – | blauviolett |
| 707 | $C_2H_5$ | $CH_3$ | Cl | $CH_3$ | – | – | blauviolett |
| 708 | $C_2H_5$ | $CH_3$ | $SO_2-CH_3$ | $CH_3$ | – | – | blau |
| 712 | $C_2H_5$ | $CH_3$ | H | $CH_3$ | $CH_3$ | $CH_3OSO_3^{\ominus}$ | rotviolett |
| 713 | $C_2H_5$ | $CH_3$ | H | $CH_2-C_6H_5$ | $CH_3$ | $CH_3OSO_3^{\ominus}$ | rotviolett |
| 715 | $C_2H_5$ | $CH_3$ | $CH_3$ | $CH_3$ | $CH_3$ | $CH_3OSO_3^{\ominus}$ | blauviolett |
| 717 | $C_2H_5$ | $CH_3$ | Cl | $CH_3$ | $CH_3$ | $CH_3OSO_3^{\ominus}$ | blau |
| 718 | $C_2H_5$ | $CH_3$ | $SO_2-CH_3$ | $CH_3$ | $CH_3$ | $CH_3OSO_3^{\ominus}$ | blau |
| 721 | 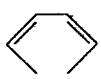 | | H | H | – | – | blauviolett |
| 722 | 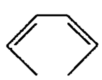 | | H | $CH_3$ | – | – | blauviolett |
| 723 | 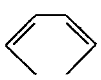 | | $CH_3$ | $CH_3$ | – | – | blauviolett |
| 724 | 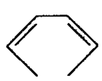 | | $SO_2CH_3$ | $CH_3$ | – | – | blau |
| 725 | 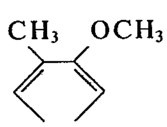 | | H | H | – | – | blau |
| 726 | desgl. | | H | $CH_3$ | – | – | blau |
| 727 | desgl. | | $CH_3$ | $CH_3$ | – | – | blau |
| 728 | desgl. | | $SO_2CH_3$ | $CH_3$ | – | – | neutralblau |
| 729 | desgl. | | Cl | $CH_3$ | – | – | neutralblau |
| 730 | 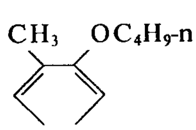 | | H | H | – | – | blau |

Fortsetzung

| Verbindung | $R_1$ | $R_2$ | $R_6$ | $R_8$ | $R_9$ | $A^{\ominus}$ | Fluoreszenzfarbe in Lösung |
|---|---|---|---|---|---|---|---|
| 731 | 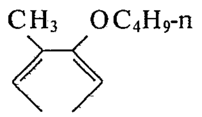 | | H | $CH_3$ | – | – | blau |
| 741 | 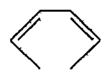 | | H | $CH_3$ | $CH_3$ | $CH_3OSO_3^{\ominus}$ | blau |
| 742 | desgl. | | $CH_3$ | $CH_3$ | $CH_3$ | $CH_3OSO_3^{\ominus}$ | blau |
| 743 | desgl. | | $SO_2CH_3$ | $CH_3$ | $CH_3$ | $CH_3OSO_3^{\ominus}$ | neutralblau |
| 744 | 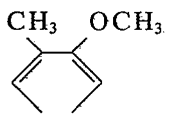 | | H | $CH_3$ | $CH_3$ | $CH_3OSO_3^{\ominus}$ | blau |
| 745 | desgl. | | $CH_3$ | $CH_3$ | $CH_3$ | $CH_3OSO_3^{\ominus}$ | blau |
| 746 | 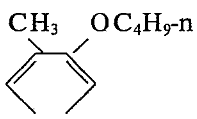 | | H | $CH_3$ | $CH_3$ | $CH_3OSO_3^{\ominus}$ | blau |

Die vorstehend aufgeführten quaternären Salze lösen sich in Wasser mit blauer bis blaugrüner Fluoreszenz am Tageslicht.

Beispiel 8

Polyacrylnitril-Textilgewebe werden im Flottenverhältnis 1 : 40 30 Minuten kochend mit einer Färbeflotte behandelt, die 0,2% der Verbindung der Formel (401), 8% Na-chlorit 50%ig, 4% Na-nitrat, 4% Chloritstabilisator (alle Prozentangaben bezogen auf das Textilmaterial) enthält und mit Ameisensäure auf pH 3,5 gestellt ist. Nach dem Spülen und Trocknen erhält man ein sehr gut und brillant aufgehelltes Polyacrylnitrilgewebe.
Ähnliche Ergebnisse erhält man, wenn man gleich wie oben beschrieben verfährt, jedoch die Verbindungen der Formeln (201), (402), (403), (404), (601), (602), (712), (715) oder (718) einsetzt.

**Patentansprüche**

1. Verbindungen der Formel

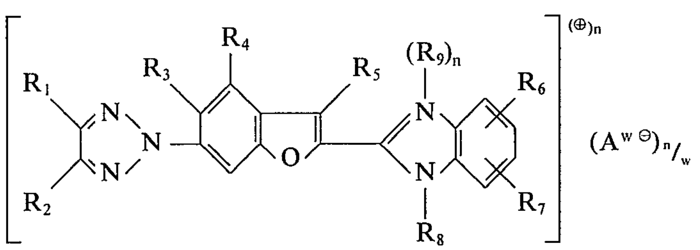

in der

$R_1$ eine Alkylgruppe mit 1 bis 12 Kohlenstoffatomen, eine Alkoxygruppe mit 1–4 Kohlenstoffatomen, einen Phenylrest, der gegebenenfalls durch Alkyl mit 1–4 C-Atomen, Phenyl, Alkoxy mit 1–4 C-Atomen oder Chlor substituiert sein kann, einen Benzylrest oder einen Styrylrest,

$R_2$ Wasserstoff, Cyan, Carboxy, $C_1$–$C_4$-Alkylcarbonylamino, Benzoylamino oder den Rest R oder

$R_1$ und $R_2$ zusammen einen ankondensierten Naphthalinring oder Benzolring, der gegebenenfalls durch Alkyl mit 1 bis 4 Kohlenstoffatomen und/oder Alkoxy mit 1 bis 4 Kohlenstoffatomen substituiert sein kann,

$R_3$ und $R_4$ unabhängig voneinander Wasserstoff, Methyl, Äthyl, Methoxy, Chlor,

$R_5$ Wasserstoff, Alkyl mit 1 bis 4 Kohlenstoffatomen, vorzugsweise Methyl, oder gegebenenfalls mit Methyl und/oder Methoxy substituiertes Phenyl,

$R_6$ Wasserstoff, Alkyl mit 1 bis 4 Kohlenstoffatomen, vorzugsweise Methyl, Methoxy, Chlor, Alkylsulfonyl mit 1 bis 4 Kohlenstoffatomen, Cyan, eine Sulfo- oder Carboxygruppe oder deren funktionelle Derivate,

$R_7$ Wasserstoff, Methyl, Methoxy oder Cl,

$R_8$ Alkyl mit 1 bis 4 Kohlenstoffatomen, vorzugsweise Methyl, Hydroxyalkyl mit 2 bis 4 Kohlenstoffatomen, Cyanethyl, Cyclohexyl, Benzyl oder gegebenenfalls durch Chlor, Methyl oder Methoxy substituiertes Phenyl,

$R_9$ gegebenenfalls mit Hydroxy oder Alkoxy mit 1 bis 4 Kohlenstoffatomen substituiertes Alkyl mit 1 bis 4 Kohlenstoffatomen, gegebenenfalls mit Chlor oder Methoxy substituiertes Benzyl oder einen Rest $-CH_2CN$, $-CH_2CONH_2$ oder $-CH_2COOR$, worin R eine Alkylgruppe mit 1 bis 4 Kohlenstoffatomen ist,

n die Zahl 0 oder 1,

$A^{\ominus}$ ein farbloses Anion und

w die Wertigkeit des Anions A

bedeuten.

2. Verbindungen gemäß Anspruch 1 der Formel

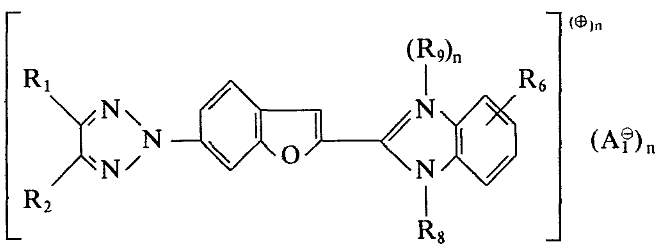

worin

$R_1$, $R_2$, $R_6$, $R_8$ und $R_9$ die in Anspruch 1 angegebene Bedeutung haben,

n die Zahl 0 oder 1 und

$A_1^{\ominus}$ ein Halogen-, Methylsulfat-, Ethylsulfat- oder p-Tolylsulfation bedeuten.

3. Verfahren zur Herstellung von Verbindungen gemäß Anspruch 1, dadurch gekennzeichnet, daß man eine Verbindung der Formel

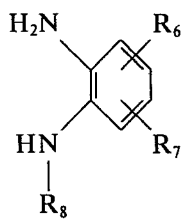

in der $R_6$, $R_7$, $R_8$ die in Anspruch 1 angegebene Bedeutung haben mit einer Verbindung der Formel

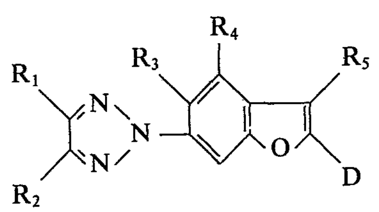

in der $R_1$, $R_2$, $R_3$, $R_4$, $R_5$ die in Anspruch 1 angegebene Bedeutung haben und D eine funktionell abgewandelte Carboxylgruppe ist, acyliert, in dem Acylierungsprodukt in an sich bekannter Weise den Imidazol-Ringschluß herbeiführt und gegebenenfalls das Reaktionsprodukt quaterniert oder protoniert.

4. Verwendung einer Verbindung gemäß den Ansprüchen 1 und 2 zum optischen Aufhellen von organischen hochmolekularen Materialien.

**Claims**

1. Compounds of the formula

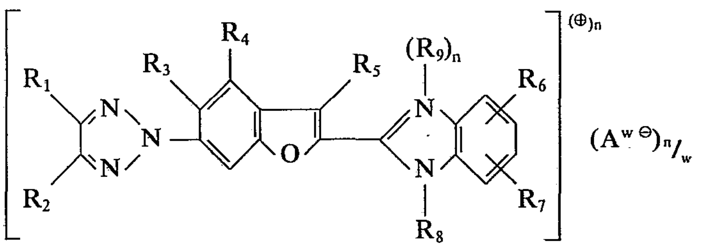

in which

$R_1$ denotes an alkyl group with 1 to 12 carbon atoms, an alkoxy group with 1—4 carbon atoms, a phenyl radical, which can optionally be substituted by alkyl with 1—4 C atoms, phenyl, alkoxy with 1—4 C atoms or chlorine, or a benzyl radical or a styryl radical,
$R_2$ denotes hydrogen, cyano, carboxyl, $C_1$—$C_4$-alkylcarbonylamino, benzoylamino or the radical R or
$R_1$ and $R_2$ together denote a fused-on naphthalene ring or benzene ring, which can optionally be substituted by alkyl with 1 to 4 carbon atoms and/or alkoxy with 1 to 4 carbon atoms,
$R_3$ and $R_4$ independently of one another denote hydrogen, methyl, ethyl, methoxy or chlorine,
$R_5$ denotes hydrogen, alkyl with 1 to 4 carbon atoms, preferably methyl, or phenyl which is optionally substituted by methyl and/or methoxy,
$R_6$ denotes hydrogen, alkyl with 1 to 4 carbon atoms, preferably methyl, methoxy, chlorine, alkylsulphonyl with 1 to 4 carbon atoms, cyano or a sulfpho or carboxyl group or functional derivates thereof,
$R_7$ denotes hydrogen, methyl, methoxy or Cl,
$R_8$ denotes alkyl with 1 to 4 carbon atoms, preferably methyl, hydroxyalkyl with 2 to 4 carbon atoms, cyanoethyl, cyclohexyl, benzyl or phenyl optionally substituted by chlorine, methyl or methoxy,
$R_9$ denotes alkyl with 1 to 4 carbon atoms, optionally substituted by hydroxyl or alkoxy with 1 to 4 carbon atoms, benzyl which is optionally substituted by chlorine or methoxy or a radical $-CH_2CN$, $-CH_2CONH_2$ or $-CH_2COOR$,

wherein

R is an alkyl group with 1 to 4 carbon atoms,
n denotes the number 0 or 1,
$A^{\ominus}$ denotes a colourless anion and
w denotes the valency of the anion A.

2. Compounds according to Claim 1, of the formula

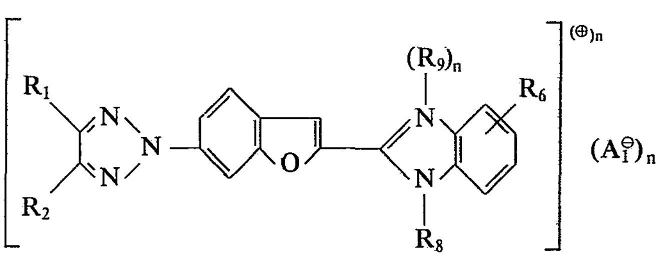

wherein

$R_1$, $R_2$, $R_6$, $R_8$ and $R_9$ have the meaning indicated in Claim 1,
n denotes the number 0 or 1 and
$A_1^{\ominus}$ denotes a halogen ion, methylsulphate ion, ethylsulphate ion or p-tolylsulphate ion.

3. Process for the preparation of compounds according to Claim 1, characterised in that a compound of the formula

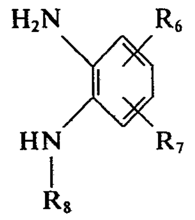

in which
$R_6$, $R_7$ and $R_8$ have the meaning indicated in Claim 1, is acylated with a compound of the formula

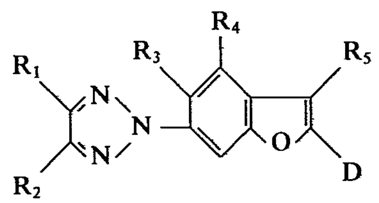

in which

$R_1$, $R_2$, $R_3$, have the meaning indicated in Claim 1 and
$R_4$ and $R_5$
D is a functionally modified carboxyl group,

the imidazole cyclisation is brought about in the acylation product in a manner which is in itself known and the reaction product is optionally quaternised or protonated.

4. Use of a compound according to Claims 1 and 2 for optically brightening organic high-molecular materials.

**Revindications**

1. Composés de formule:

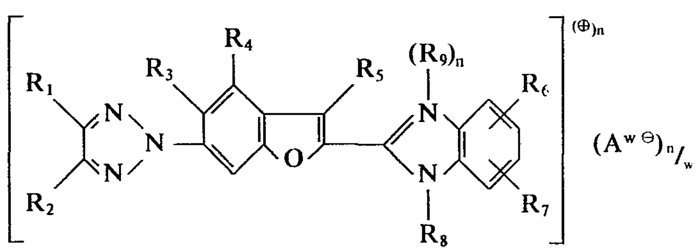

dans laquelle

- $R_1$ représente un groupe alcoyle ayant 1 à 12 atomes de carbone, un groupe alcoxy ayant 1 à 4 atomes de carbone, un radical phényle qui peut être éventuellement substitué par un alcoyle ayant 1 à 4 atomes de carbone, phényle, alcoxy ayant 1 à 4 atomes de carbone ou chlore, un radical benzyle ou un radical styryle,
- $R_2$ de l'hydrogène, cyano, carboxy, alcoyl($C_1$—$C_4$)carbonylamino, benzoylamino ou le radical R, ou
- $R_1$ et $R_2$ sont ensemble un noyau naphthalène ou noyau benzénique accolé qui peut être éventuellement substitué par un alcoyle ayant 1 à 4 atomes de carbone et/ou alcoxy 1 à 4 atomes de carbone,
- $R_3$ et $R_4$ indépendamment l'un de l'autre représentent de l'hydrogène, méthyle, éthyle, méthoxy, chlore,
- $R_5$ de l'hydrogène, un alcoyle ayant 1 à 4 atomes de carbone, de préférence méthyle, ou un phényle éventuellement substitué par un méthyle et/ou méthoxy,

$R_6$ de l'hydrogène, un alcoyle ayant 1 à 4 atomes de carbone, de préférence méthyle, méthoxy, chlore, alcoylsulfonyle ayant 1 à 4 atomes de carbone, cyano, un groupe sulfo ou carboxy ou leurs dérivés fonctionnels,

$R_7$ de l'hydrogène, méthyle, méthoxy ou Cl,

$R_8$ un alcoyle ayant 1 à 4 atomes de carbone, de préférence méthyle, hydroxy-alcoyle ayant 2 à 4 atomes de carbone, cyanoéthyle, cyclohexyle, benzyle ou phényle éventuellement substitué par du chlore, méthyle ou méthoxy,

$R_9$ un alcoyle ayant 1 à 4 atomes de carbone éventuellement substitué par un hydroxy ou alcoxy ayant 1 à 4 atomes de carbone, un benzyle éventuellement substitué par du chlore ou méthoxy ou un radical $-CH_2CN$, $-CH_2CONH_2$ ou $-CH_2COOR$, où R est un groupe alcoyle ayant 1 à 4 atomes de carbone,

n le nombre 0 ou 1,

$A^{\ominus}$ un anion incolore et

w la valence de l'anion A.

2. Composés selon la revendication 1, de formule:

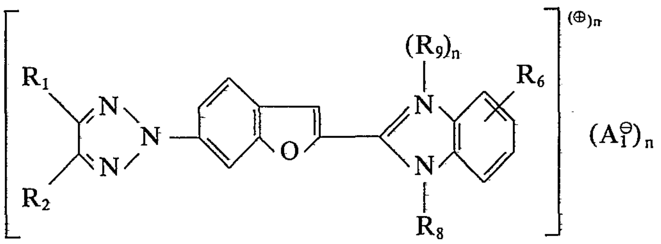

dans laquelle

$R_1$, $R_2$, $R_6$, $R_8$ et $R_9$ ont la signification indiquée à la revendication 1,

n est le nombre 0 ou 1 et

$A_1^-$ un ion halogène, méthylsulfate, éthylsulfate ou p-tolylsulfate.

3. Procédé de préparation de composés selon la revendication 1, caractérisé en ce qu'on acyle un composé de formule:

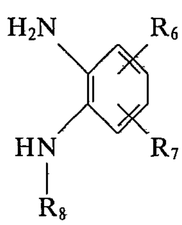

dans laquelle $R_6$, $R_7$, $R_8$ ont la signification indiquée à la revendication 1, avec un composé de formule:

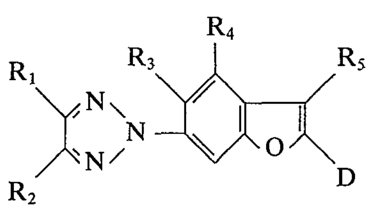

dans laquelle $R_1$, $R_2$, $R_3$, $R_4$, $R_5$ ont la signification indiquée à la revendication 1 et D est un groupe carboxyle fonctionnellement modifié, on produit dans le produit d'acylation d'une manière connue en elle-même la cyclisation imidazol et éventuellement on quaternise ou protonise le produit de réaction.

4. Utilisation d'une composé selon les revendications 1 et 2 pour l'éclaircissement optique de matériaux organiques à poids moléculaire élevé.